# EUROPEAN PATENT SPECIFICATION

(11) **EP 0 654 481 B1**
(45) Date of publication and mention of the grant of the patent: **13.02.2002**
(21) Application number: 94118059.8
(22) Date of filing: 15.11.1994
(51) Int. Cl.: C07K 5/06, A23L 1/236, F26B 17/12

(54) **Process for producing alpha-L-aspartyl-L-phenylalanine methyl ester**
Verfahren zur Herstellung von alpha-L-Aspartyl-L-Phenylalaninmethylester
Procédé pour la préparation de alpha-L-aspartyl-L-phenylalanine methyl ester

(30) Priority: 19.11.1993 JP 29045393
(43) Date of publication of application: 24.05.1995
(73) Proprietor: Ajinomoto Co., Inc., Tokyo 104 (JP)
(72) Inventor: Hirano, Atsuhiko, Central Res. Lab., Kawasaki-shi, Kanagawa-ken (JP); Kataoka, Takehiko, Central Res. Lab., Kawasaki-shi, Kanagawa-ken (JP); Kishimoto, Shinichi, Central Res. Lab., Kawasaki-shi, Kanagawa-ken (JP); Yamahra, Satoshi, Tokai Plant, Yokkaichi-shi, Mie-ken (JP)
(74) Representative: Strehl Schübel-Hopf & Partner

(56) References cited:
- EP-A- 0 255 092
- EP-A- 0 256 515

## Description

### Field of the Invention

This invention relates to a significantly improved process for producing α-L-aspartyl-L-phenylalanine methyl ester (hereinafter referred to as α-APM).

α-APM is expected to be widely utilized as a low caloric dietary sweetener which is about 200 times as sweet as sugar.

### Description of the Prior Art

The final product α-APM is generally in the form of dried powder or granule. Processes for producing granules include, for example, a method wherein wet extrusion granulation is carried out before drying (Japanese Patent Application Laid-open No. 95862-1984), a method wherein dry compression granulation is carried out after drying (Japanese Patent Publication No. 15268-1989). Various means for drying such as flush drying, fluidized drying, drying under reduced pressure may be employed.

EP-A-256 515 discloses a method for drying AMP crystals using a gas having a defined humidity.

EP-A 255 092 discloses a method for drying AMP crystals involving different temperatures.

The water content of α-APM after drying is desirably not more than 5% (% hereinafter used means that on wet basis), considering adhesion to the apparatus or wall of the pipe during subsequent transporting and packaging steps. However, the particular value may considerably vary depending on means and conditions of drying. Drying steps conducted under the same drying conditions may often provide dried products with different water content. For example, when the wet crystal cake obtained by crystallization is crushed and subjected to fluidized drying, it is inevitable that the wet crystal cake is unevenly crushed and unevenly dried depending on the unevenness of crushing, providing a variation of the water content of about 1 - 2 % in the final dried product. To avoid such problems, multi-step crushing and grinding of the crystals may be conducted as the drying degree proceeds so that the crystals should be uniformly dried to have a water content around the desired value. However, determination of conditions and conducting such multi-step operations are often very complicated.

Moreover, the water content of the dried product is known to vary considerably depending on the heat load, i.e., the amount of the wet crystals to be dried. For example, when wet crystals of α-APM with a water content of 25 % are continuously flush dried using hot air at 170 °C, the amount of the supplied wet crystals is reduced to half for convenience of the preceding step. As the result, we found that the water content of the dried product is significantly reduced, i.e., from about 3.5 to about 1.6 %.

Such variation becomes a problem particularly when the water content of the final dried product is fixed within a limited range. For example, in the above flush drying, the dried product with a water content reduced to 1.6 % due to variation of load should be redissolved, and again subjected to the steps from recrystallization to drying when the standard of water content is restricted to 3 - 4 %. Therefore operations which are not essential are required and the equipment availability is reduced, which is one of the main reasons of an increase in cost of the production. To avoid such problems, the drying conditions such as amount and temperature of the hot air should be changed according to the change of the load. It requires much labor to determine such conditions.

When the standard of the water content of the dried product is fixed to the defined value or lower, the manufacturers so eagerly try not to produce products beyond the above standard that they employ too much drying strength during the drying step. As the result, overdried products with relatively low water content are often evenly obtained. Considering the stability of the crystals and DKP production during storage of the dried product, in addition to the adhesion of the dried product in the steps after drying, the water content of the dried α-APM is desirably within the range of 2.5 to 4 %. It is not desirable to evenly obtain overdried products with relatively low water content. Further, α-APM tends to produce IIB crystals with poor solubility upon overdrying. Accordingly, to avoid loss due to the production of a product below the standard, and further to enhance easiness of handling and storage stability as well as solubility of the dried product, it is important to control the water content of the dried α-APM to a fixed value.

### Problem to be solved by the Invention

The object of the invention is to develop a process for stably producing α-APM with controlled water content.

### Means to solve the Problem

The present inventors have studied intensively to solve the above problems. As the result they have obtained the surprising finding, that α-APM with controlled water content can be stably produced by firstly drying wet crystals of α-APM obtained by crystallization, then contacting the product obtained by such drying step with an air flow at a fixed temperature and humidity. That is, if the temperature and humidity of the environment is known under which the equilibrium water content of the particular crystal form is in agreement with the objective water content, an evenly dried product with a water content within a wide range can be converted, in a short time, to a humidity-adjusted product having the desirable water content by passing a gas adjusted to said temperature and said humidity.

A thus obtained humidity-adjusted product, when stored in an environment whose equilibrium humidity is different from the water content of said product, can retain its water content for a long period of time as long as it is appropriately packaged.

The present inventors have succeeded in solving the above problems and stabilizing steps by applying such findings to the practical process for producing α-APM. Thus we have attained the present invention.

The present invention is directed to a process for producing α-L-aspartyl-L-phenylalanine methyl ester which is characterized by drying wet crystals of α-L-aspartyl-L-phenylalanine methyl ester obtained by crystallization to adjust the water content to not more than 5% (wet basis), and contacting the dried crystals with an air flow adjusted to a temperature of 20 - 80 °C and a relative humidity of 20 - 90 RH% to control the water content.

The crystal form of α-APM after drying includes, for example, I type, II type (Japanese Patent Application Laid-open No. 172444-1984). The equilibrium water content at the temperature and humidity of the environment vary depending, for example, on the particular crystal form. Accordingly, it is impossible to fix the temperature and humidity of the air to be contacted with the dried α-APM at a specified value. However, lower temperature and lower humidity requires longer contacting time because the change of water content of the contacted α-APM crystals becomes slower. Higher temperature and higher humidity are accompanied by considerable generation of DKP during the contact, which can not be disregarded and results in problems in quality. Desirably, the temperature and humidity is within the range of 20 - 80 °C and 20 - 90 RH%, more desirably within the range of 30 - 60 °C and 30 - 90 RH%, respectively. By using a gas adjusted within the above range, the water content of the dried product of α-APM can be controlled to the desired value within a short time, i.e., in an hour. Further, if α-APM after drying contains IIB type crystals, they can be readily converted to IIA type crystals during control of water content. Accordingly, the above problem of solubility can be solved.

As the gas to be contacted with the dried α-APM, air is advantageously used considering cost. Inert gas or a mixture of air and inert gas may be used. Inert gas used herein means a gas with low reactivity with α-APM within the above temperature and humidity range. The example includes, for example, nitrogen, carbon dioxide, helium, argon and the like.

The form of the dried product to be contacted with the gas of which temperature and humidity are adjusted includes, for example, powder, granules, crushed cake and the like. To attain uniform contact with the gas, it is desirably in the form of powder or granules.

As the contacting device, generally used solid-gas contacting devices, such as fluid bed device and flush devices can be used. Especially, a fluid bed device, which has provided a high contact efficiency of α-APM crystals and a gas, can be used to shorten the contact time required to obtain the desired water content, resulting in minimizing the equipment required for treatment of the specified amount of α-APM. The operation may be either a continuous or a batch process. The average contacting time of the dried α-APM and the gas corresponds to the average residence time in a continuous process and draft time in a batch process. When a fluid bed device is used, the suitable value of the gas flow varies depending on the form and amount of the dried product to be charged. It is not necessarily more than minimal fluidizing speed. It is suitably in the range from 0.1 to 4 m/sec (as a linear velocity) for a charged amount of 10 - 200 kg/m² per unit fluidized bed.

### Advantages of the Invention

According to the present process, even when the water content of α-APM immediately after drying varies, a humidity-adjusted product with a fixed water content can be stably obtained by an industrial scale operation. As the result, complicated examination steps to determine the drying conditions can be eliminated, and further dried products below standards do not have to be redissolved and recycled to the main step, resulting in fewer operation steps and enhanced availability of equipment. Accordingly, this process is practically valuable because the steps are rationalized.

The present invention will be illustrated in detail in the following examples.

### Example 1

Wet crystals obtained by the crystallization of α-APM were subjected to fluidized drying at 60°C to obtain two types of α-APM powders having different water contents ((A) 2.6 % and (B) 3.5 %). The crystal forms of (A) and (B) are completely IB. The sample (A) (250 g) was charged in a fluid bed device (fluidized bed area, 0.0028 m²), and fluidized by passing a gas adjusted to an inlet temperature of 40 °C and relative humidity of 50 RH% at a linear velocity of 0.4 m/sec. Such fluidizing and humidity adjustment operations are carried out for 10 minutes. The same operations were carried out for sample B. The water content of the samples (A) and (B) after treatment, measured by Karl Fischer's method, was in the range of 3.1 - 3.2 %. As the result of X-ray powder diffraction measurement, no change in crystal form of sample was observed after humidity adjustment.

### Example 2

Powders of α-APM mainly containing IB crystals with 10 % of IIA (water content: (A) 2.5 %; (B) 3.4 %) obtained by flush drying were compressed and granulated. A fluid bed device with fluidized bed area of 0.5 m² was used for granulated samples (A) and (B), and fluidizing and humidity-adjustment operations were continuously conducted. The operation conditions were as follows: Air (inlet temperature, 40 °C; relative humidity, 40 RH%) supplied at a linear velocity of 0.4 m/sec.; Feed of the sample, 180 kg/h; residence time of the sample in the device, 10 min.). The water content of the humidity adjusted product, measured according to Karl Fischer's method, was within the range of 3.2 - 3.3 %. As the result of X-ray powder diffraction measurement, no change was observed in crystal form.

## Claims

1. A process for producing α-L-aspartyl-L-phenylalanine methyl ester having a controlled water content which comprises drying wet crystals of α-L-aspartyl-L-phenylalanine methyl ester obtained upon crystallization to adjust the water content to not more than 5 % (wet basis), followed by contacting the said dried crystals with a gas having temperature adjusted to 20 - 80 °C and a relative humidity adjusted to 20 - 90 RH% to control the water content of the crystals.

2. A process according to claim 1, wherein the inlet temperature and relative humidity of the gas to be contacted with α-L-aspartyl-L-phenylalanine methyl ester are within the range of 30 - 60 °C and 30 - 90 RH%, respectively.

3. A process according to claim 1 or 2, wherein a fluid apparatus is employed to contact dried α-L-aspartyl-L-phenylalanine methyl ester with a gas of which temperature and humidity are adjusted.

4. A process according to claim 3, wherein the amount of α-L-aspartyl-L-phenylalanine methyl ester to be supplied to the fluid apparatus is within the range of 10 - 200 kg/m² per unit area of the fluidized bed, and the linear velocity of the gas to be supplied is within the range of 0.1 - 4.0 m/sec.

5. A process according to any one of claims 1 to 4, wherein the average time for the contact between dried α-L-aspartyl-L-phenylalanine methyl ester and the temperature- and humidity-adjusted gas is within an hour.

6. A process according to any one of claims 1 to 5, wherein the α-L-aspartyl-L-phenylalanine methyl ester to be contacted with temperature- and humidity-adjusted gas is in the form of powder or granules.

7. A process according to any one of claims 1 to 6, wherein the crystal form of α-L-aspartyl-L-phenylalanine methyl ester to be contacted with temperature- and humidity adjusted gas is type I or II, or a mixture thereof.

8. A process according to claim 7, wherein the type II crystals of α-L-aspartyl-L-phenylalanine methyl ester to be contacted with temperature- and humidity-adjusted gas are type IIB or a mixture of IIA and IIB.

9. A process according to any one of claims 1 to 8, wherein the gas to be contacted with dried α-L-aspartyl-L-phenylalanine methyl ester is air or an inert gas, or a mixture thereof.

10. A process according to any one of claims 1 to 8, wherein the gas to be contacted with dried a-L-aspartyl-L-phenylalanine methyl ester is air.

## Patentansprüche

1. Verfahren zur Herstellung von α-L-Aspartyl-L-phenylalaninmethylester mit kontrolliertem Wassergehalt, welches das Trocknen von feuchten Kristallen von α-L-Aspartyl-L-phenylalaninmethylester, erhalten durch Kristallistion, unter Einstellung des Wassergehalts auf nicht mehr als 5 % (Feuchtbasis), und das nachfolgende Kontaktieren der getrockneten Kristalle mit einem Gas einer Temperatur, die auf 20 - 80 °C eingestellt ist, und einer relativen Feuchtigkeit, die auf 20 - 90 RH% eingestellt ist, zur Kontrolle des Wassergehalts der Kristalle umfasst.

2. Verfahren nach Anspruch 1, wobei die Einlasstemperatur und die relative Feuchtigkeit des mit α-L-Aspartyl-L-phenylalaninmethylester zu kontaktierenden Gases innerhalb des Bereiches von 30 - 60 °C beziehungsweise 30 - 90 RH% liegen.

3. Verfahren nach Anspruch 1 oder 2, wobei eine Flüssigkeitsvorrichtung eingesetzt wird, um den getrockneten α-L-Aspartyl-L-phenylalaninemethylester mit einem Gas zu kontaktieren, dessen Temperatur und Feuchtigkeit eingestellt sind.

4. Verfahren nach Anspruch 3, wobei die Menge des α-L-Aspartyl-L-phenylalaninmethylesters, die in die Flüssigkeitsvorrichtung gegeben werden soll, innerhalb des Bereiches von 10 - 200 kg/m² pro Flächeneinheit des Fließbetts liegt und die lineare Geschwindigkeit des zuzugebenden Gases innerhalb des Bereichs von 0,1 bis 4,0 m/s liegt.

5. Verfahren nach einem der Ansprüche 1 bis 4, wobei die mittlere Dauer für den Kontakt zwischem dem getrockeneten α-L-Aspartyl-L-phenylalaninmethylester und dem hinsichtlich der Temperatur und des Feuchtigkeit eingestellten Gas innerhalb einer Stunde liegt.

6. Verfahren nach einem der Ansprüche 1 bis 5, wobei der α-L-Aspartyl-L-phenylalaninmethylester, der mit dem hinsichtlich der Temperatur und der Feuchtigkeit eingestellten Gas kontaktiert werden soll, in der Form eines Pulvers oder eines Granulats vorliegt.

7. Verfahren nach einem der Ansprüche 1 bis 6, wobei die Kristallform von α-L-Aspartyl-L-phenylalaninmethylester, der mit dem hinsichtlich der Temperatur und des Feuchtigkeit eingestellten Gas kontaktiert werden soll, vom Typ I oder II ist oder ein Gemisch derselben ist.

8. Verfahren nach Anspruch 7, wobei die Typ II-Kristalle von α-L-Aspartyl-L-phenylalaninmethylester, die mit dem hinsichtlich der Temperatur und der Feuchtigkeit eingestellten Gas kontaktiert werden sollen, vom Typ IIB sind oder ein Gemisch von IIA und IIB sind.

9. Verfahren nach einem der Ansprüche 1 bis 8, wobei das mit dem getrockneten α-L-Aspartyl-L-phenylalaninmethylester zu kontaktierende Gas Luft oder ein Inertgas oder ein Gemisch derselben ist.

10. Verfahren nach einem der Ansprüche 1 bis 8, wobei das mit dem getrockneten α-L-Aspartyl-L-phenylalaninmethylester zu kontaktierende Gas Luft ist.

## Revendications

1. Procédé pour la production de l'ester méthylique de la α-L-aspartyl-L-phénylalanine ayant une teneur en eau contrôlée, qui comprend le séchage des cristaux humides de l'ester méthylique de la a-L-aspartyl-L-phénylalanine obtenus par cristallisation afin d'ajuster la teneur en eau à une valeur non supérieure à 5% (sur une base humide), suivi par la mise en contact desdits cristaux séchés avec un gaz ayant une température ajustée à 20 à 80°C et une humidité relative ajustée à 20 à 90% d'HR afin de contrôler la teneur en eau des cristaux.

2. Procédé selon la revendication 1, dans lequel la température d'entrée et l'humidité relative du gaz que l'on veut mettre en contact avec l'ester méthylique de la α-L-aspartyl-L-phénylalanhe sont respectivement dans l'intervalle de 30 à 60°C et de 30 à 90% d'HR.

3. Procédé selon la revendication 1 ou 2, dans lequel on utilise un appareil fluidisé pour mettre en contact l'ester méthylique de la α-L-aspartyl-L-phénylalanine séché avec un gaz dont la température et l'humidité sont ajustées.

4. Procédé selon la revendication 3, dans lequel la quantité d'ester méthylique de la α-L-aspartyl-L-phénylalanine à alimenter dans l'appareil fluidisé est dans l'intervalle de 10 à 200 kg/m² par surface unitaire du lit fluidisé et la vitesse linéaire du gaz à alimenter est dans l'intervalle de 0,1 à 4,0 m/seconde.

5. Procédé selon l'une quelconque des revendications 1 à 4, dans lequel le temps moyen pour le contact entre l'ester méthylique de la α-L-aspartyl-L-phénylalanine séché et le gaz ayant une température et une humidité ajustées est d'environ une heure.

6. Procédé selon l'une quelconque des revendications 1 à 5, dans lequel l'ester méthylique de la α-L-aspartyl-L-phénylalanine que l'on veut mettre en contact avec le gaz ayant une température et une humidité ajustées est sous la forme d'une poudre ou de granulés.

7. Procédé selon l'une quelconque des revendications 1 à 6, dans lequel la forme cristalline de l'ester méthylique de la α-L-aspartyl-L-phénylalanine que l'on veut mettre en contact avec le gaz ayant une température et une humidité ajustées est de type I ou II ou un de leurs mélanges.

8. Procédé selon la revendication 7, dans lequel les cristaux de type II d'ester méthylique de la α-L-aspartyl-L-phénylalanine que l'on veut mettre en contact avec le gaz ayant une température et une humidité ajustées sont de type IIB ou un mélange de IIA et IIB.

9. Procédé selon l'une quelconque des revendications 1 à 8, dans lequel le gaz que l'on veut mettre en contact avec l'ester méthylique de la α-L-aspartyl-L-phénylalanine séché est de l'air ou un gaz inerte ou un de leurs mélanges.

10. Procédé selon l'une quelconque des revendications 1 à 8, dans lequel le gaz que l'on veut mettre en contact avec l'ester méthylique de la α-L-aspartyl-L-phénylalanine séché est de l'air.
